# EUROPEAN PATENT APPLICATION

(11) **EP 3 173 066 A1**
(43) Date of publication of application: **31.05.2017**
(21) Application number: 15003363.7
(22) Date of filing: 25.11.2015
(51) Int. Cl.: A61K 9/00, A61K 45/06, A61K 9/70, A61K 33/06

(54) **PLASTER COMPRISING ANTIEDEMATOUS SUBSTANCES FOR THE TREATMENT OF BRUISING AND INJURY**

(71) Applicant: Kubica, Krzysztof, 43-450 Ustron (PL); Taciak, Przemyslaw, 08-124 Mokobody (PL); Mazurek, Adam, 01-518 Warszawa (PL)
(72) Inventor: Kubica, Krzysztof, 43-450 Ustron (PL); Taciak, Przemyslaw, 08-124 Mokobody (PL); Mazurek, Adam, 01-518 Warszawa (PL)
(74) Representative: Patpol Kancelaria Patentowa Sp. z o.o.

(57) **Abstract**

The invention relates to an innovative form of antiedematous substance application, in a form of plasters, comprising aluminum acetate-tartrate.

Apart from aluminum acetate-tartrate (or another substance mentioned above), the plaster can comprise one or few other active substances having an adjunctive or enhancing effect with respect to aluminum acetate-tartrate. The plaster structure is based on a possibility of storing and controlled release of the biologically active substances. The active substances, depending to the plaster structure, may be subject to the modified (e.g. sustained) release. Therapeutic properties of the product result from the combination of all constituents of the given product. Analysis has also shown a synergistic effect.

## Description

The invention relates to an innovative form of antiedematous substance application, in a form of plasters comprising aluminum acetate-tartrate. The formulation can comprise other substances employed in the treatment of edemas and bruising such as: troxerutin, oxerutin, aescin (or an extract of horse chestnut seeds), heparin, diosmin (or an extract of the Chinese tea plant), allantoin (or an extract of bean and/or soy and/or horse chestnut and/or comfrey roots), an arnica (*arnica montana*) extract, as well as combinations thereof.

In addition to aluminum acetate-tartrate (or another above-mentioned substance) the plaster can comprise one or more of additional adjunctive or enhancing active substances for aluminum acetate tartrate. A structure of the plaster is based on a possibility of storing and controlled release of the biologically active substances. The active substances, depending to the plaster structure, may be subject to the modified (e.g. sustained) release. Therapeutic properties of the product result from the combination of all constituents of the given product. Analyses also showed a synergy of activities in the case of several products. Moreover, within the framework of the present patent application, the analysis was carried out with respect to the use of combinations of substances with antiedematous activities in the innovative plaster form, wherein said substances comprised the following biologically active substances: blood-flow enhancing substances, blood-vessel sealing substances, disinfecting substances, astringent substances, osteoarticular pain alleviating substances, substances correcting the function and structure of the skin and/or osteoarticular system, cooling substances, warming substances, substances that are conventionally used in diseases related to osteoarticular system and skin, muscle-relaxing substances, exsiccating substances, moisture-absorbing substances, defatting substances, and substances reducing an allergic reaction.

By using the innovative form of the plaster with aluminum acetate-tartrate, the beneficial therapeutic effect of said substances was maximized, with simultaneous reduction of adverse and toxic reactions. An unquestionable advantage of using the developed form is convenience and ease of application by the patient.

### STATE OF THE ART

A plaster, generally, is a piece of fabric coated with a self-adhesive substance, applied i.a. on wounds. Plasters of various sizes are commercially available, most often with a dressing, as well as plasters in a form of several-meter long wound roll. They have a protective function and preclude a contact of the wound with the external media. In the product of the invention, functionality of the plaster is extended by employing it as a carrier of a biologically active substance. This results in a much easier application combined with maintaining dosage repeatability, which is very hard to achieve in the case of semi-solid drug formulation deposited from a collapsible tube. At the same time, the plaster retains its protective function, while additionally preventing adsorption of the formulation through the external fabric. The product of the invention enables rapid, repeatable application, wherein there is no need to wait for absorption of the formulation. Since the active substance is released directly from the plaster, there are no losses associated with rubbing-in the semi-solid drug form. Due to placing the drug substance in the plaster-integrated carrier, uniformity of distribution of the substance at the therapeutic surface is also obtained, which is in practice unobtainable with the semi-solid drug forms. In the case of plasters from which the active agent is controllably released, dosage stability is also attained, which results in a possibility of reaching a uniform therapeutic concentration of the active substance.

Aluminum acetate-tartrate is an astringent active substance, which reduces tissue edema and causes pain decrease within the inflammatory region. After application onto the skin, aluminum acetate-tartrate causes protein denaturation within oozing and inflammatory skin lesions, with resultant reduction of exudation and inflammatory condition. The same applies also to sensory-nerve endings in the skin, which results in amelioration of the pain within the post-traumatic inflammatory region. In the case of aluminum acetate-tartrate solutions, concentration of 1 - 3 % is mostly used. In a pre-clinical study on rabbits, repeated application of 2% solution did not provoke irritation. Aluminum contained in the therapeutic agents applied onto the undamaged skin is absorbed in 0,012% only and is rapidly excreted from the body.

Aluminum acetate-tartrate is recommended in the case of: post-traumatic edemas, sprain, bruising, trauma resulting injury, contusion, inflammatory conditions of muscles, tendons, joints, skin, particularly accompanied by edema, joint, tendon and muscle injury produced by excessive exercise, inflammatory skin reactions caused by tanning, inflammatory skin reactions caused by insect bites. It is applied externally as a solution or gel, 3-4 times a day onto aching or inflammatorily altered sites. The formulation can be employed also as a compress. For sensitive skin application of0,75% cream it is suggested. It should not be applied at oozing skin lesions and open wounds. Placement of the injury-afflicted limb in the higher position will assure good outflow of the venous blood, lymph and inter-tissue fluids. Cooling of the skin, most often by applying compresses (cooling compresses) with water, reduces blood flow and results in contraction of vessels.

Examples of products on the Polish market, where aluminum acetate-tartrate is used as the active agent include:

| **Formulation name** | **Form** | **Dose** | **Manufacturer** |
|---|---|---|---|
| Altacet | Tablet (6 x 1g) | 1 tablet contains: 1g of aluminum acetate tartrate, 70 mg of boric acid | Sandoz GmbH |
| Altacet | Gel (40 g or 75 g) | 1 g of gel contains 10 mg of aluminum acetate tartrate | EMO-FARM |
| Altaziaja | Gel (75g) | 1 g of gel contains 10 mg of aluminum acetate tartrate | Ziaja |
| Plyn Burowa | Liquid (100 ml) | 7,5-9% solution of monobasic aluminum acetate | Hasco-Lek; AMARA |
| Kamagel | Gel (40g) | 1 g of gel contains: 0,05 g of aluminum acetate tartrate as a 10% solution and 0,01 g of liquid glycolic extract of chamomile flowers in a 1:1 ratio | KRKA |

The described method of the invention also contemplates the use of ointments, pastes, creams, suspensions, emulsions, granulate, as well as microforms such as liposomes, niosomes, micelles, nanospheres, carbon nanotubes, polymersomes, nanocapsules and microemulsions in the formulation as carriers for the therapeutic substances. Other compounds can also be used in the formulation. The carriers used make up the integral part of the plaster. Use of various carriers for the active substance enables modification of its release.

Taking into account all of the aforementioned properties, the novel plasters exhibit many advantages over the formulations employed so far, in particular with respect to effectiveness of dosing, dosage control, adverse reactions, toxic effects, duration of activity, comfort of use and economy.

### EXPERIMENTAL PART

Aluminum acetate-tartrate is a substance of astringent (coagulates proteins at the tissue surface), analgesic and anti-inflammatory activity. It is applied topically in the case of edemas and bruising of the soft tissue. It is employed as a gel or tablets (to be dissolved in water). Plasters with aluminum acetate-tartrate are characterized by considerably more direct application, more precise dosage and longer duration of activity, which translates into the more efficient therapy. Prototype plasters contain the aluminum acetate-tartrate solution - 0,1 g of the 2 percent solution or gel per 1 cm² of the therapeutic surface of the plaster, which corresponds to 2 mg of aluminum acetate-tartrate per 1 cm² of the therapeutic surface of the plaster (assuming the density of 1 g/cm³).

The method of the invention provides combining of an aqueous solution, gel, hydrogel, ointment, paste, cream, suspension, emulsion, granulate, liposomes, niosomes, micelles, nanospheres, polymersomes, nanocapsules and/or microemulsion of aluminum acetate-tartrate with an innovative carrier (e.g.: a polyurethane foam, as well as cotton, flax, silk, polycondensation plastics, oxidised cellulose, gelatine sponge, collagen film, fibrinous plastic) embedded in a silicone frame (or a frame made of another material which meets suitable requirements). The whole is covered with a material, which restricts contact of the formulation with external media, and at the same time enables air and water vapor flow. The performed analysis shows that the obtained formulation exhibited significantly increased therapeutic efficiency.

The prototypes were subjected to the following tests:
1. examination of theoretical density
2. examination of sorptive capacity
3. examination of wash-out resistance
4. examination of pharmaceutical availability
5. examination of permeability for gases
6. examination of thermal insulation property

Plasters with aluminum acetate-tartrate, troxerutin, oxerutin, aescin, heparin, diosmin, allantoin and an arnica extract were analyzed. The best properties were shown by a plaster with aluminum acetate-tartrate as an active substance.

The method according to the invention also provides structural innovations of the inventive product, that make it possible for the plaster to adapt its shape to the contact surface of the active substance with a specific joint or site of the body. Said operation would enable more effective action of the drug substance. In addition, one of the plaster properties is that it acquires a rigid structure with the intended shape, which forces optimal position of the body.

The method according to the invention was described in detail in reference to aluminum acetate-tartrate. The formulation may contain other substances used in the treatment of edemas and bruises such as: troxerutin, oxerutin, aescin (or an extract of horse chestnut seeds), heparin, diosmin (or an extract of Chinese tea plant), allantoin (or an extract of bean and/or soy and/or horse chestnut and/or comfrey roots), an arnica (*arnica montana*) extract, as well as combinations thereof.

Prototypes were also studied, which contain more than one active substance. The product comprising one or more of antiedematous substances in combination with blood-flow enhancing substances, i.a. diosmin, provided better absorption of the active substance.

The product comprising one or more of antiedematous substances in combination with blood-vessel sealing substances, i.a. rutin, provided synergetic antiedematous activity.

The product comprising one or more of antiedematous substances in combination with disinfecting substances, i.a. octenidine, provided beneficial therapeutic activity in the case of injuries with potential bacterial infection, by inhibiting the microbial growth.

The product comprising one or more of antiedematous substances in combination with astringent substances, i.a. tannin, provided synergetic antiedematous activity.

The product comprising one or more of antiedematous substances in combination with osteoarticular pain alleviating substances i.a. naproxen, provided beneficial activity in the case of the treatment of edemas with concomitant osteoarticular pain.

The product comprising one or more of antiedematous substances in combination with substances correcting skin and/or osteoarticular system function and structure, i.a. glycolic acid, accelerated regeneration of the skin cells.

The product comprising one or more of antiedematous substances in combination with cooling substances, i.a. menthol, provided the synergetic antiedematous activity and cooling effect.

The product comprising one or more of antiedematous substances in combination with warming substances, i. a. capsaicin, provided better absorption of the active substance and the warming effect.

The product comprising one or more of antiedematous substances in combination with substances conventionally used in the osteoarticular system diseases, i.a. aescin, provided the synergetic antiedematous activity.

The product comprising one or more of antiedematous substances in combination with exsiccating substances, i.a. zinc oxide, provided the synergetic antiedematous activity.

The product comprising one or more of antiedematous substances in combination with allergic-reaction reducing substances, i.a. acetylsalicylic acid, provided accelerated recovery of the tissue to the physiological state.

In addition to the active substance, the product can additionally comprise adjunctive or enhancing substances:
- Exsiccating, water-absorbing substances such as: zinc oxide, kaolin, burnt magnesia, *calcium carbonicum*, colloidal silica.
- substances enhancing skin-adhesion and coating such as: talc, magnesium stearate, zinc stearate, colloidal silica, zinc oxide, titanium oxide.
- Astringent substances such as: tannin, tannalbin, monobasic bismuth nitrate, zinc oxide, tanning agents, aluminum potassium sulfate, lime water, bismuth tribromophenolate, monobasic bismuth carbonate, aluminum hydroxide, silver compounds, gallic acid.
- Emollient substances such as: glycerol, ethylene glycol, propylene glycol, glycerol acetate (mono-, di- and triacetin), butyl lactate, butyl myristate, butyl oleate, butyl palmitate, butyl stearate.
- Preservatives such as: methyl hydroxybenzoate, ethyl hydroxybenzoate, propyl hydroxybenzoate, benzalkonium chloride, benzalkonium benzalkonium bromide, boric acid, parahydroxybenzoic acid esters, benzoic acid and salts thereof, formic acid and salts thereof, propionic acid and salts thereof, sorbic acid and salts thereof, benzyl alcohol, chlorhexidine salts, parachlorometacresol, phenoxyethanol,
- Antioxidant substances such as: sodium sulfate, potassium sulfate, sodium bisulfate, potassium bisulfate, pyrosulfates, hyposulfites, tocopherols, nordihydroguaiaretic acid, ascorbyl myristate, ascorbyl palmitate, ascorbyl stearate, propyl gallate, octyl palmitate, dodecyl gallate, butylhydroxyanisole, tocopherol, ascorbic acid, quercetin, rosemary acid, flavonoids, synthetic antioxidants (BHA, BHT)
- Gelling, and gel-enhancing substances such as: gelatin, methylcellulose, hydroxyethylcellulose, hydroxypropylcellulose, hydroxypropylmethylcellulose, poly(acrylic acid), diethanolamine, triethanolamine, propylene glycol, agar
- Resorption-enhancing substances such as: dimethylsulfoxide, urea, hyaluronic acid.
- Humectant substances such as: glycerol, sorbitol, propylene glycol, polyoxyethylene glycols of the molecular weight less than 600, glucose, mannitol.
- Viscosity-enhancing substances such as: alginic acid and salts thereof, propylene glycol alginate, pectins, tragacanth, gum acacia, agar, carrageen, carboxymethylcellulose sodium salt, colloidal microcrystalline cellulose, polyacrylic acid, gelatin, lecithyns, microcrystalline cellulose, methylcellulose, hydroxyethylcellulose, hydroxypropylcellulose, hydroxypropylmethylcellulose, polyoxyethylene glycols, polyvinylpyrrolidone, polyvinyl alcohol, colloidal silica, aluminum stearate, calcium stearate, magnesium stearate, cetyl alcohol, wax.
- Surfactants such as: soaps, alkylsulfates, alkylsulfonates, cholic acid salts, benzalkonium chloride, benzalkonium bromide, lauryl alcohol, cetyl alcohol, cetostearyl alcohol, cholesterol, white wax, yellow wax, spermaceti, cetyl palmitate, glycerol monostearate, sorbitan esters of higher fatty acids, polyoxyethylenesorbitan esters of higher fatty acids, esters of higher fatty acids and polyoxyethyleneglycol glycols, esters of sucrose and fatty acids, esters of higher fatty alcohols with polyoxyethylene glycols, polyoxyethylenepolyoxypropylene glycols, gum acacia, tragacanth, pectins, alginates, methylcellulose, polyvinylpyrrolidone, carbomer, proteins, bentonite, alumina, magnesium carbonate, silica, cetylsulfate sodium, cholesterol, monoglyceride, tween, span, cetylsulfonate sodium, dioctylsulfonosuccinate sodium, cetylpyridinium bromide
- Absorption promoters such as: ethyl alcohol, phosphatides, cholic acid salts, propylene glycol, urea, sulfoxides, pyrrolidones, alcohols and fatty acids and esters thereof, laurocapram, amides and terpenes and derivatives thereof, dimethylformamide,
- Solvents such as: water, ethyl alcohol.
- Cooling substances such as: menthol and derivatives thereof, isopulegol, carvone, cubebol, D-bornyl, L-bornyl, exonorbornyl, 2-methylisobornyl, 2-ethylfenchyl, 2-methylbornyl, cis-pinan-2-yl, verbanyl, isobornyl and other fenchyl derivatives
- Warming substances such as: capsaicin, vanillyl ethers, gingerol, piperines, paprika oil, ginger oil, Japanese pepper and black pepper extract, camphor, belladonna root extract
- Antioxidative efficacy enhancing substances such as: edetic acid and salts thereof.
- Flavor-enhancing substances such as: peppermint oil, lavender oil, coriander oil, anise oil
- Humidifying-lubricating-emollient substances such as: fatty acids and esters and amides thereof, fatty alcohols and esters thereof, oxyalkylated fatty alcohols, perhydrosqualene, sheep wool fats, PCL (oil-gland secretion of aquatic birds), lecithin, mucus-rich plant material
- Vitamins such as: vitamin C, vitamin B₃, vitamin B₅, vitaminised oils
- Amino acids as well as polypeptides composed of amino acids with a long-chain structure such as: keratin, elastin, collagen
- Ceramides
- Exfoliating substances, skin appearance enhancing substances such as:
   glycolic acid, lactic acid, malic acid, citric acid
- Peeling agents such as: comminuted nutshells and stones, chololiths, sugar, hydrogenated jojoba oil, polyethylene particles
- Film-forming substances such as: methacrylic acid esters and polymers thereof, polyvinyl chloride, nitrocellulose, shellac, wax, resins, silicones, polyvinyl alcohol, polyvinylpyrrolidone, ethylene oxide condensation products
- Sun filters such as: para-aminobenzoic acid esters, benzophenone derivatives, quinin, salicylic acid, salol, antipyrine, cyclic ketones, cinnamate compounds
- Sunscreens such as: zinc oxide, titanium oxide, talc, magnesium oxide, calcium carbonate, barium salts, bentonite
- Antiseptic substances such as: dichlorophenol, hexachlorophenol, hexylresorcinol, chloramines, iodophors, silver compounds (silver nitrate), triphenylmetane dyes with gelatin or albumin, gentian, acridine dyes (e.g. rivanol), potassium hypermanganate, ethanol, propanol, isopropanol, biphenylol, phenylphenol, chlorhexidine digluconate, hydrogen peroxide, iodine (e.g. tincture of iodine, povidone iodine), octenidine, Peruvian balsam, ichthyol
- Adhesives such as: rubber homogeneously mixed with resins, rosin, waxes, fats, zinc oxide and other auxiliary substances, copolymers of acrylic acid and esters thereof or other polymers
- Analgesic substances such as: acetylsalicylic acid, indometacin, ibuprofen, naproxen, nabumeton, meloxicam, nimesulide, celecoxib, alcofenac, mesalazine, sodium salicylate, choline salicylate, fenclofenac, sulindac, tolmetin, piprofen, fenbufen, indoprofen, flurbiprofen, oxaprozin, mefenamic acid, niflumic acid, meclofenamic acid, flufenamic acid, etodolac, ketorolac, propyphenazone, metamizole, phenylbutazone, clofenazone, azoprenazone, piroxicam, sudoxicam, isoxicam, tenoxicam, lornoxicam, meloxicam, rofecoxib, etoricoxib, valdecoxib, tenidap, diacerein, paracetamol, methyl salicylate
- Blood-vessel sealing substances, such as: rutin, aescin, vitamin K, vitamin C, witch hazel extract, ginkgo extract (or substances isolated from ginkgo or the ginkgo herb), devil's claw extract, arnica extract, camomile extract, diosmin, ruscogenin (butcher's-broom extract)
- Essential oils such as: ambrette seed oil, anise oil, angelica oil, basil oil, bergamot oil, birch oil, cedar oil, cinnamon oil, citric oil, savory oil, eucalyptus oil, geranium oil, clove oil, hash oil, ginger oil, orris oil, juniper oil, fir oil, camphor oil, cardamom oil, cumin oil, coriander oil, dill oil, lavandin oil, lavender oil, melissa oil, peppermint oil, almond oil, nutmeg oil, marigold oil, rose oil, camomile oil, sandalwood oil, pine oil, spike oil, sage oil, turpentine oil, thyme oil.

## Claims

1. A disposable plaster for reducing enema, comprising a layer with an appropriate concentration of the antiedematous substance(s) and having an appropriate shape, which is adapted to the therapeutic surface, **characterized in that**
a. it contains in the internal portion thereof a layer with the antiedematous substance(s) in a form of a solution, gel, suspension, ointment, cream, dusting powder, powder, liposomes, nanosomes;
b. the content of antiedematous substances is from 0,001% to 75% with respect to the total carrier.

2. The plaster according to claim 1, **characterized in that** the formulation can comprise the following substances employed in the treatment of edemas and bruising: aluminum acetate-tartrate, troxerutin, oxerutin, aescin (or an extract of horse chestnut seeds), heparin, diosmin (or an extract of Chinese tea plant), allantoin (or an extract of bean and/or soy and/or horse chestnut and/or comfrey roots), an arnica (*arnica montana*) extract, and combinations thereof.

3. The plaster according to claim 1, **characterized in that** it can comprise antiedematous substances with auxiliary substances modifying their therapeutic properties and/or enhancing action thereof, including: blood-flow enhancing substances, i.a. diosmin, blood-vessel sealing substances, i.a. rutin, disinfecting substances, i.a. octenidine, astringent substances, i.a. tannin, osteoarticular pain alleviating substances i.a. naproxen, skin and/or substances correcting osteoarticular system function and structure, i.a. glycolic acid, cooling substances, i.a. menthol, warming substances, i.a. capsaicin, substances conventionally used in osteoarticular system diseases, i.a. aescin, exsiccating substances, i.a. zinc oxide, allergic-reaction reducing substances, i.a. acetylsalicylic acid.

4. The plaster according to claim 1, **characterized in that** it can possess a stiffened structure, which enables movement restriction in the given site.

5. The plaster according to claim 1, **characterized in that** it can possess a flexible structure, which enables to adapt the plaster to the body surface.
